# EUROPEAN PATENT SPECIFICATION

(11) **EP 0 340 793 B1**
(45) Date of publication and mention of the grant of the patent: **30.08.1995**
(21) Application number: 89108162.2
(22) Date of filing: 05.05.1989
(51) Int. Cl.: C12N 5/10, A61K 39/00, A61K 35/26

(54) **Endowing cells with antibody specificity**
Zellen mit ausgestattener Antikörperspezifität
Cellules dotées avec spécifité d'anticorps

(30) Priority: 04.05.1988 IL 86278
(43) Date of publication of application: 08.11.1989
(73) Proprietor: YEDA RESEARCH AND DEVELOPMENT COMPANY LIMITED, Rehovot 76100 (IL)
(72) Inventor: Eshhar, Zelig, Rehovot (IL); Gross, Gideon, Doar Korazin 12340 (IL); Waks, Tova, Petah Tikva (IL)
(74) Representative: VOSSIUS & PARTNER

(56) References cited:
- EP-A- 0 180 878
- WO-A-86/01533
- BIOCHEMICAL AND BIOPHYSICAL RESEARCH COMMUNICATIONS vol. 149, no. 3, 31December 1987, New York US pages 960 - 968; Yoshihisa Kuwana et al.:"Expression of chimeric receptor composed of immunoglobulin-derived V regionsand T-cell receptor-derived C regions"
- SCHWEIZERISCHE MEDIZINISCHE WOCHENSCHRIFT vol. 116, no. 43, 1986, Basel CHpages 1459 - 1464; J.Cline: "Gene therapy:current status and future directions"
- NATURE vol. 320, 10 April 1986, London GB pages 549 - 551; C.T.Denny et al.: "Achromosome 14 inversion in a T-cell lymphoma is caused by site specificrecombination between immunoglobulin and T-cell receptor loci"
- CELL vol. 42, August 1985, New York US pages 81 - 87; Jordi Yagüe et al.: "TheT-cell receptor:the alfa and beta chains define idotype,and antigen and MHC specificty"
- CELL vol. 60, no. 6, 23 March 1990, New York US pages 929 - 939; J.Goverman etal.: "Chimeric immunoglobulin-T cell receptor proteins form functionalreceptors:implications for T cell receptor complex formation and activation"
- Immunological Rev.,1983,vol.72, Berke
- Mechanisms of cell mediated cytotoxity,edited by Clark and Goldstein,Plenum Publishing Corporation, 1982, Berke
- Adv.Expt.Biol.Med. 1982,Clark et al.
- Immunological Reviews,1987,No.95,Jenkins et al.
- Science,vol.248,1990,Schwartz
- Cell,vol.71,1992,Schwartz
- Cellular Immunology,vol.95,1985,Tirosh et al.
- The Journal of Immunology,vol.139,1987Ostergaard et al.
- Nature vol.350,1987,Trenn et al.
- J.Exp.Med,vol.166,1987,Ding-Young et al.
- Cell,vol.68,1992,Romeo et al.
- The Journal of Immunology,vol.150,No.12,1993,Wacholtz et al.

## Description

The invention relates to a process for the generation of lymphocytes transfected with one or two expression vectors containing a chimeric T-cell receptor (TcR) gene. As set out in the following there was constructed a model system which comprises expression vectors which were transfected and which were functionally expressed in T-killer cells.
Because of the restrictions imposed by corecognition of self MHC plus antigen, the acquisition of new specificity by grafting of TcR genes is limited to experimentations either in vitro or in vivo in inbred combinations. Such manipulations are practically impossible in an outbred population.

Unlike antibodies, the T cells recognize antigens only in association with products of the major histocompatibility complex (MHC). Such dual recognition is mediated by combination of the variable regions of both the α and β chains that comprise the TcR. Recently it became possible to endow T cells with a given specificity by DNA mediated transfer of cloned genes coding for the α and β TcR chains (Dembic et al. Nature 320 232-238 (1986)). In general, the expression as a dimer of both α and β chains of a given TcR has been required in order to display a defined specificity although it has been implied that the Vβ is responsible for the MHC specificity (Saito et al. Nature 329 256-259 (1987)).

In order to overcome the limitations set out above, and in order a be able to design at will T-cells having a desired predetermined specificty, there were constructed T-cells with antibody-type specificity. The invention is applicable to a wide variety of cells as set out herein.
Based on the extensive degree of similarity in structure and organization between the antibody and TcR molecules, it was assumed that it will be possible to replace the V-region of TcR with an antibody's one in a manner that will result in a functional chimeric TcR. According to the invention it is possible to construct and functionally express chimeric TcR that recognize antigen in non-MHC restricted manner and effectively transmit transmembrane signal for T cell activation.
According to the invention there were constructed chimeric T cell receptor genes by recombining the V_{H} and V_{L} gene segments of an anti-TNP antibody with the constant region exons of the T cell receptor's (TcR) α and β chain. Following transfection into cytotoxic hybridomas, expression of a novel functional T cell receptor was detected. The chimeric receptor manifested the idiotype of the antibody and endowed the T cells with non-MHC restricted, anti-TNP reactivity. This model system demonstrates that chimeric TcR with an antibody-like binding site can be designed and functionally expressed in a variety of T-cells. This manipulation offers a novel approach for engineering at will T-cells of any desired specificity, provided that such specificity can be predefined by monoclonal antibodies. The various aspects of this invention are suitable gene pairs for introduction and expression in certain vectors, the use of such vectors for transfecting cells (T-cells and others, as defined) in order to endow them with a predetermined antigenic specificity. The invention further relates to pharmaceutical compositions for the prevention and cure of certain diseases. Other aspects will become clear hereinafter.
As stated above the invention relates to lymphocytes, comprising populations of T-killer cells, T-helper cells, T-suppressor cells, lymphokine activated cells, natural killer cells and the like. As stated above the invention uses a recombinant gene-pair adapted to endow mononuclear cells with antibody-type specificity, where the genes are:
a. gene segments containing exons coding for the constant region of the T-cell receptor ( α, β, γ, δ, chains),
b. gene-segments containing the leader and rearranged variable-joining exons encoding for a specific antibody's heavy and light chains, the said mononuclear cells being lymphocytes, comprising populations of T-killer cells, T-helper cells, T-suppressor cells, lymphokine activated cells, natural killer cells, and the like.

The above gene segment can be genomic or cDNA segments.
According to a preferred aspect the invention uses a gene pair with gene segments coding for the constant region of α or β chains, each of which being ligated with either the rearranged variable gene segment of the antibody's heavy or light chain and vice versa, it being clear that the T-cell receptor can be of the same species as the antibody or of a different species. Preferably the rearranged gene segments code for a binding site of an antibody molecule either from the same species of the T-cell receptor gene or another species where the rearranged gene segments code for antibody specific towards tumor-specific antigens, tumor-associated antigens, viral antigens, modified self-antigens, parasitic antigens, bacteria antigens, fungal antigens, autoimmune disease type antigens, or other foreign antigens. According to a further embodiment the rearranged gene segments code for monoclonal antibodies reacting with a defined type of tumor cells. The invention further relates to expression vectors for the efficient transfection of a cell type, comprising a recombinant gene pair as defined above. Such a vector can be a plasmid backbone containing promoter, polyadenylation site and drug selection markers. Preferred vectors according to the invention are the plasmids pRSV2 and pRSV3. There is preferably used a pair of expression vectors in which one vector comprises a plasmid with one selection marker (say neo^{R}) into which a rearranged gene segment coding for the variable region of the antibody's light chain together with the gene segment coding for the constant region of the T-cell receptor's α or β chains are cloned, and the second vector comprises another selection marker (say gpt) into which a rearranged gene segment coding for the variable region of the antibody's heavy chain together with the gene segment of the constant region of the complementary T-cell receptor's chain ( β or α) used in the first vector is cloned.
The invention further relates to mononuclear cells containing a chimeric pair of genes as defined above.
Yet another aspect of the invention relates to a composition for treating a patient in need thereof comprising as active ingredient lymphocytes which have been taken from the patient, propagated in vitro, which have been transfected with a gene pair as above described or with a vector as defined above.
For producing expression vectors used in the invention and for constructing a gene pair a hybridoma producing a monoclonal antibody for the desired antigen has been selected, a genomic library from the restricted DNA fragments that contain the rearranged V_{L} and V_{H} exons has been constructed, the clones carrying full length rearranged V_{L} and V_{H} exons have been isolated and therefrom, the DNA segments containing the leader exon and the rearranged VDJ_{H} and VJ_{L} exons have been isolated and each of them has been subcloned into an expression vector containing either the neo^{R} or gpt selection marker followed by inserting into each of the same vectors, 3' to the VDJ_{H} or VJ_{L} either one of the genomic fragments containing all of the constant region exons and 5' flanking untranslated regions of the T-cell receptor α and β chains isolated from an embryonic DNA library, resulting in a set of chimeric genes comprised of V_{H}C_{α}, V_{L}C_{β}, V_{L}C_{α}, V_{H}C_{β} each of which is cloned in one of the expression vectors containing either the neo^{R} or gpt selection markers.
One aspect of the invention relates to a process where a combination of two plasmids is used for the transfection of the T-cell, one of which comprises the variable region of the light chain and the constant region of either α or β; the other the variable region of the heavy chain with the constant region of the α- or β chain.
The following example is a model experiment which demonstrates the feasibility of the above general principles and their wide scope of applicability. The invention is not restricted to this specific embodiment.

### Example

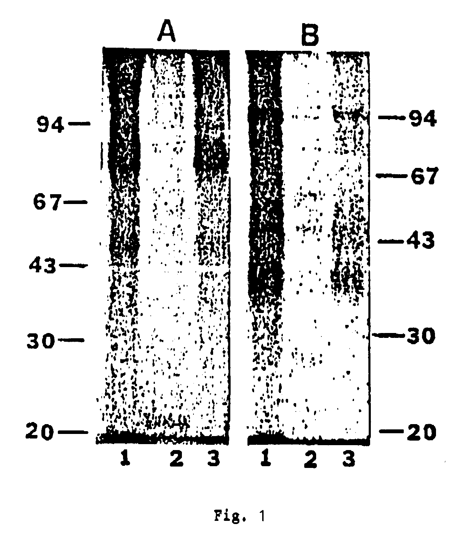

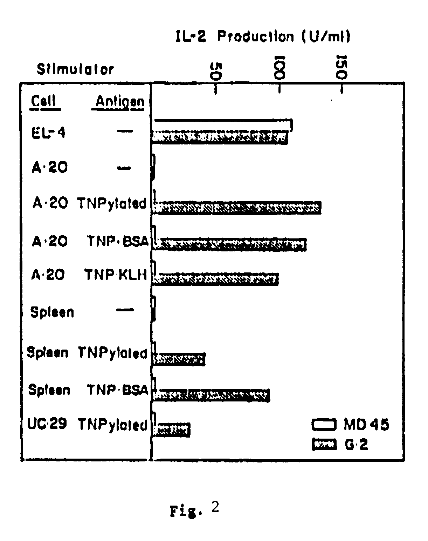

To construct the chimeric TcR genes we ligated genomic segments each one containing the rearranged V_{J} and leader exons of either heavy or light chain of the Sp6 anti-TNP IgM antibody (Ochi et al. Proc. Natl. Acad. Sci. USA 80 6351-6355 (1983)) with constant region exons of either the α or β chains of the TcR. The chimeric genes were inserted into pRSV expression vector containing the Rous sarcoma promotor and either the Neo^{R} or the gpt drug resistance genes. By protoplast fusion we transfected each of the vectors into MD.45 - a CTL hybridoma of BALB/c origin that can be stimulated by H-2D^{b} cells both for IL-2 production and specific killing of target cells (Kaufmann et al. Proc. Natl. Acad. Sci. USA 78 2502-2507 (1981)). Out of the drug resistant transfectants we selected the hybridomas that expressed the chimeric gene (using V_{H} and V_{L} probes). The clone producing the highest levels of one chain, was retransfected with the construct containing the complementary chain and the other drug marker. Double transfectants that grew in the presence of both mycophenolic acid and G.418 were checked by Northern analysis for the transcription of both chimeric genes and by immunoblotting and immunoprecipitation for the expression of the Sp6 idiotype (using the 20.5 mAb). The functional expression of the chimeric receptor was evaluated by the ability of the cells to respond by IL-2 production to TNPylated cells of various origins and by TNP-protein antigens either alone or presented by different cells.
Following DNA transfer of the chimeric genes combination of either V_{L}C_{α} + V_{H}C_{β} or V_{L}C_{β} + V_{H}C_{α} into the MD.45 CTL hybridoma, almost all the transfectants expressed both complementary chains of 1.8 Kb for V_{L}C_{α} or _{β} and 1.9 Kb for V_{H}C_{α} or _{β} chains (Gross et al., Transp. Proc. 21, 127 - 130 (1989)). The expression of the chimeric polypeptide was analyzed by immunoblotting of cell lysates using anti-Sp6 idiotype mAb 20.5 or by immunoprecipitation using the 20.5 mAb and anti-TcR β 8.3 subgroup F23.1 mAb (Fig. 1). Under non-reducing conditions the reaction with anti-id and anti-β TcR resulted in a broad band of apparent molecular weight of 80 Kd that is composed of two bands of 83 Kd and 77 Kd. In some transfectants a band of 42-45 Kd was also apparent. After reduction, however, the idiotypic determinant recognized by 20.5 mAb in the immunoblot was destroyed and the 80 Kd complex was dissociated into the 42 Kd polypeptide. Interestingly, transfectants that received either the V_{H}C_{α} or V_{H}C_{β} alone also expressed the 83 Kd complex as well as the 42 Kd chain carrying the 20.5 idiotype. Considering that MD.45 hybridoma expresses its α β dimer (Becker et al. Nature 316 606-619 (1985)) and only the β chain of BW 5147 fusion partner (Lustgarten and Eshhar, unpublished data), together with the fact that the 20.5 idiotope (as well as the anti-TNP binding site) is expressed solely on V_{H} Sp6 and is sensitive to denaturation under reducing conditions, we can conclude that in transfectants that had received the V_{H}C_{α} or V_{H}C_{β} gene, a chimeric chain is expressed that can form functional dimer with the autologous complementary α or β TcR chains. The chains in part of the dimers are not linked by disulfide bonds and therefore migrate as single band in SDS-PAGE. The double transfectants most likely express on their surface in addition to the V_{L}C_{α} - V_{H}C_{β} (or V_{L}C_{β} -V_{H}C_{α} ) chimeric receptor dimers, also the heterodimers that result from various combinations of pairing of the chimeric chain with the complementary autologous α and β chains. This results in the broad band observed in the immunoprecipitation of surface iodinated TcR by either anti-id or anti-TcR (Fig. 1).
In order to study whether the chimeric receptor preserved the antibody's anti-TNP specificity and the ability to transmit transmembrane signal for T cell activation, we coincubated the transfectants with different stimulator cells either TNPylated or in the presence of various TNP-protein antigens. Fig. 2 shows the degree of IL-2 production by G.2 - one of the transfectants that received V_{L}C_{β} + V_{H}C_{α} chimeric chains. G.2 expressed on its surface both the autologous TcR as evidenced by its reactivity (like the parental MD.45 hybridoma) toward EL-4 stimulator cells. Unlike MD.45, G.2 underwent triggering by TNP covalently coupled to A.20 (BALB/c B lymphoma) or UC.29 (human B lymphoblastoid). In addition TNPylated proteins (such as TNP-BSA, TNP-KLH and others), could stimulate IL-2 production by G.2 either in a soluble form and even better in the presence of BALB/c spleen cells or A.20 cells that are known to be good antigen presenters. Interestingly, the transfectants that received only the V_{H}C_{α} or the V_{H}C_{β} chimeric gene and expressed the Sp6 idiotype, could also respond to TNP indicating that the V_{H} of Sp6 contains all the information needed to construct the TNP-binding site.
Taken together, our results clearly demonstrate that it is possible to construct, transfect and functionally express chimeric T cell genes that manifest antibody specificity. This novel approach should be extendable to enable the engineering at will of the specificity of T cells in non-MHC restricted manner, in such a way that a given set of genes could be transferred to T cells of any origin. Such T cells could then be returned to their donor and manifest the acquired specificity. Following such manipulation, the cells acquire a new specificity encoded by the chimeric genes that is of antibody-type, i.e., not restricted by self-MHC molecules.
The results obtained demonstrate that in a similar manner it is possible to prepare a wide variety of pairs of such chimeric genes encoding chimeric receptors that are directed to various target antigens which are predefined by specific monoclonal antibodies. Such antigens can be those found in tumor cells of a certain cancer, viral antigens, modified self antigens, antigens of bacteria, parasites, fungi, antigens of autoimmune diseases, and any other antigens of which directing cellular immune responses can benefit the patient.
It is one of the advantages of this invention that it enables taking the patient's own cells, their propagation in vitro, to select (if needed) a certain effector subpopulation (killers, helper, or suppressor cells), and to direct the desired specificity of such cells by introducing into them the pair of engineered chimeric genes. Such cells, upon reimplantation into the patient, will function against the target antigens as dictated by the chimeric genes.

## Claims

1. A mononuclear T-killer cell or natural killer cell which expresses a chimeric receptor which permits said cell to selectively kill target cells bearing a predefined target antigen in a non-MHC restricted manner, said cell containing a recombinant gene encoding said chimeric receptor, said gene comprising:
(a) gene segments containing coding sequences coding for the constant region of the T-cell receptor (TCR) (α, β, γ and δ chains), and
(b) gene-segments containing coding sequences coding for the leader, the variable and joining subregions of the rearranged variable region of the heavy or light chain of a specific antibody.

2. The T-killer cell according to claim 1, wherein said gene comprises:
a pair of recombinant genes, wherein the first set of gene segments coding for the constant region of the T-cell receptor's α or β chain is recombined with either the gene segments of the rearranged variable region of the antibody's heavy chain or of the antibody's light chain, and wherein the second set of gene segments coding for the constant region of the T-cell receptor's β or α chain is recombined with the gene segments of the rearranged variable region of the other chain of the antibody.

3. The T-killer cell according to claim 1 or 2, wherein the TCR and the antibody from which said recombinant gene or gene pair is obtained are of the same species.

4. The T-killer cell according to any one of claims 1 to 3, wherein the TCR and the antibody from which said recombinant gene or gene pair is obtained are of a different species.

5. The T-killer cell according to any one of claims 1 to 4, wherein said gene comprises a recombinant gene or a gene pair, wherein the gene segments of the rearranged variable regions code for the binding site of an antibody molecule of either the same species as the T-cell receptor or of another species.

6. The T-killer cell according to any one of claims 1 to 5, wherein said gene comprises a recombinant gene or a gene pair, wherein the gene segments of the rearranged variable regions code for an antibody with specificity to tumor-specific antigens, tumor-associated antigens, viral antigens, modified self-antigens, parasitic antigens, bacterial antigens, fungal antigens, autoimmune disease type antigens or for other foreign antigens.

7. The T-killer cell according to claim 6, wherein said gene comprises a recombinant gene or gene pair, wherein the gene segments of the rearranged variable regions code for monoclonal antibodies reacting with a defined type of tumor cells.

8. A pharmaceutical composition comprising as an active ingredient an effective quantity of T-killer cells according to any one of claims 1 to 7.

9. The pharmaceutical composition according to claim 8, wherein the T-killer cells have been taken from a patient who is to be treated with the modified T-killer cells by reimplantation.

## Patentansprüche

1. Mononucleäre T-Killerzelle oder natürliche Killerzelle, die einen chimären Rezeptor exprimiert, der es der Zelle erlaubt, Zielzellen, die ein vorbestimmtes Ziel-Antigen tragen, in einer nicht MHC-beschränkten Weise selektiv abzutöten, wobei die Zelle ein rekombinantes, den chimären Rezeptor codierendes Gen umfaßt, wobei das Gen
(a) Genabschnitte, enthaltend codierende Sequenzen, die den konstanten Bereich des T-Zellrezeptors (TCR) (α-, β-,γ- und δ-Ketten) codieren, und
(b) Genabschnitte, enthaltend codierende Sequenzen, die die Leadersequenz, die variablen und verbindenden (joining) Unterbereiche des rearrangierten variablen Bereichs der schweren oder leichten Kette eines spezifischen Antikörpers codieren,
umfaßt.

2. T-Killerzelle nach Anspruch 1, wobei das Gen ein Paar rekombinanter Gene umfaßt, wobei der erste Satz von Genabschnitten, die den konstanten Bereich der α- oder β-Kette des T-Zellrezeptors codieren, entweder mit den Genabschnitten des rearrangierten variablen Bereichs der schweren oder der leichten Kette des Antikörpers rekombiniert ist, und wobei der zweite Satz von Genabschnitten, die den konstanten Bereich der β- oder α-Kette des T-Zellrezeptors codieren, mit den Genabschnitten des rearrangierten variablen Bereichs der anderen Kette des Antikörpers rekombiniert ist.

3. T-Killerzelle nach Anspruch 1 oder 2, wobei der TCR und der Antikörper, von denen man das rekombinante Gen oder Genpaar erhält, von der gleichen Spezies stammen.

4. T-Killerzelle nach einem der Ansprüche 1 bis 3, wobei der TCR und der Antikörper, von denen man das rekombinante Gen oder Genpaar erhält, von einer unterschiedlichen Spezies stammen.

5. T-Killerzelle nach einem der Ansprüche 1 bis 4, wobei das Gen ein rekombinantes Gen oder ein Genpaar umfaßt, wobei die Genabschnitte der rearrangierten variablen Bereiche die Bindungsstelle eines Antikörpermoleküls codieren, das entweder von der gleichen Spezies wie der T-Zellrezeptor oder von einer anderen Spezies stammt.

6. T-Killerzelle nach einem der Ansprüche 1 bis 5, wobei das Gen ein rekombinantes Gen oder ein Genpaar umfaßt, wobei die Genabschnitte der rearrangierten variablen Bereiche einen Antikörper codieren, der eine Spezifität für tumorspezifische Antigene, tumorassoziierte Antigene, virale Antigene, modifizierte Eigen-Antigene, Parasitenantigene, Bakterienantigene, Pilzantigene, für Autoimmunerkrankungen typische Antigene oder andere fremde Antigene aufweist.

7. T-Killerzelle nach Anspruch 6, wobei das Gen ein rekombinantes Gen oder ein Genpaar umfaßt, wobei die Genabschnitte der rearrangierten variablen Bereiche monoclonale Antikörper codieren, die mit einem definierten Typ von Tumorzellen reagieren.

8. Arzneimittel, das als Wirkstoff eine wirksame Menge von T-Killerzellen nach einem der Ansprüche 1 bis 7 umfaßt.

9. Arzneimittel nach Anspruch 8, wobei die T-Killerzellen einem Patienten entnommen wurden, der mit den modifizierten T-Killerzellen mittels Re-Implantation behandelt werden soll.

## Revendications

1. Cellule T-tueuse mononucléaire ou cellule tueuse naturelle exprimant un récepteur chimérique qui permet à ladite cellule de tuer sélectivement des cellules-cibles portant un antigène cible prédéfini d'une façon restrictive non-MHC, ladite cellule contenant un gène recombinant codant pour ledit récepteur chimérique, ledit gène comprenant :
(a) des segments de gène contenant des séquences codantes codant pour la région constante du récepteur de cellule T (TCR) (chaînes α, β, γ et δ), et
(b) des segments de gène contenant des séquences codantes codant pour les sous-régions leader, variable et jonction de la région variable réarrangée de la chaîne lourde ou légère d'un anticorps spécifique.

2. Cellule T-tueuse suivant la revendication 1, dans laquelle ledit gène comprend une paire de gènes recombinants, où le premier jeu de segments de gène contenant des séquences codant pour la région constante de la chaîne α ou β du récepteur de cellule T est recombinée avec les segments de gène de la région variable réarrangée de la chaîne lourde de l'anticorps ou de la chaîne légère de l'anticorps, et où le second jeu de segments de gène contenant des séquences codant pour la région constante de la chaîne β ou α du récepteur de cellule T est recombinée avec les segments de gène de la région variable réarrangée de l'autre chaîne de l'anticorps.

3. Cellule T-tueuse suivant la revendication 1 ou 2, dans laquelle le TCR et l'anticorps, d'où est issu ledit gène recombinant ou ladite paire de gènes, sont de la même espèce.

4. Cellule T-tueuse suivant la revendication 1 ou 2, dans laquelle le TCR et l'anticorps, d'où est issu ledit gène recombinant ou ladite paire de gènes, sont d'une espèce différente.

5. Cellule T-tueuse suivant l'une quelconque des revendications 1 à 4, dans laquelle ledit gène comprend un gène recombinant ou une paire de gènes, où les segments de gène des régions variables réarrangées codent pour le site de liaison d'une molécule anticorps soit de la même espèce que le récepteur de cellule T soit d'une autre espèce.

6. Cellule T-tueuse suivant l'une quelconque des revendications 1 à 5, dans laquelle ledit gène comprend un gène recombinant ou une paire de gènes, où les segments de gène des régions variables réarrangées codent pour un anticorps présentant une spécificité vis-à-vis des antigènes spécifiques de tumeur, cellule T-tueuse associés à une tumeur, des antigènes viraux, des autoantigènes modifiés, des antigènes parasitaires, des antigènes bactériens, des antigènes fongiques, des antigènes de maladie auto-immune ou de tous autres antigènes étrangers.

7. Cellule T-tueuse suivant la revendication 6, dans laquelle ledit gène comprend un gène recombinant ou une paire de gènes, où les segments de gène des régions variables réarrangées codent pour des anticorps monoclonaux réagissant avec un type défini de cellules tumorales.

8. Composition thérapeutique comprenant une quantité efficace de cellules T-tueuses suivant l'une quelconque des revendications 1 à 7, en tant qu'ingrédient actif.

9. Composition thérapeutique suivant la revendication 8, dans laquelle les cellules T-tueuses ont été prélevées chez un patient qui a été traité avec les cellules T-tueuses modifiées par réimplantation.
